Europäisches Patentamt

European Patent Office

Office européen des brevets

(19)

(11) Veröffentlichungsnummer: **0 322 549**

**A2**

(12) **EUROPÄISCHE PATENTANMELDUNG**

(21) Anmeldenummer: 88119002.9

(22) Anmeldetag: 15.11.88

(51) Int. Cl.4: **G01N 33/569 , //C12Q1/04**

Die Bezeichnung der Erfindung wurde geändert (Richtlinien für die Prüfung im EPA, A-III, 7.3).

(30) Priorität: 27.11.87 CH 4616/87

(43) Veröffentlichungstag der Anmeldung:
05.07.89 Patentblatt 89/27

(84) Benannte Vertragsstaaten:
**CH DE FR GB IT LI NL**

(71) Anmelder: **F. HOFFMANN-LA ROCHE & CO. Aktiengesellschaft**

**CH-4002 Basel(CH)**

(72) Erfinder: **Müller, Hanne-Lene, Prof. Dr.**
**Amselstrasse 10**
**CH-4104 Oberwil(CH)**

(74) Vertreter: **Kloter, Rudolf et al**
**Postfach 3255 Grenzacherstrasse 124**
**CH-4002 Basel(CH)**

(54) **Verfahren zur immunologischen Bestimmung von Polysaccharid-Antigenen aus Bakterien oder Pilzen in einer biologischen Probe.**

(57) Es wird ein Verfahren zur Freisetzung von Polysaccharid-Antigenen aus Bakterien oder Pilzen in biologischen Proben oder in angezüchteten Kulturen dieser Proben beschrieben. In den allermeisten Fällen ist eine derartige Freisetzung notwendig, damit anschliessend die Polysaccharid-Antigene in an sich bekannter Weise immunologisch bestimmt werden können. Diese Freisetzung erfolgt dadurch, dass man die biologische Probe oder die angezüchtete Kultur mit einer wässrigen Phenollösung behandelt. Zweckmässigerweise wird eine 1-10%ige Phenollösung verwendet, wobei eine 2-5%ige Phenollösung bevorzugt und eine 2%ige Phenollösung besonders bevorzugt ist. Man lässt die Phenollösung während 5-30, vorzugsweise 15 Minuten auf die Probe bzw. Kultur einwirken. Zur anschliessenden immunologischen Bestimmung Polysaccharide ist keine Extraktion notwendig.

EP 0 322 549 A2

## Bestimmmung von Antigenen

Zur immunologischen Bestimmung von Polysaccharid-Antigenen aus Bakterien oder Pilzen in biologischen Proben oder in angezüchteten Kulturen dieser Proben ist in den allermeisten Fällen eine Vorbehandlung zur Freisetzung der Antigene notwendig.

Für diese Freisetzung der Antigene werden in der Praxis diverse Methoden verwendet, nämlich das Erhitzen auf 100°C, eine Enzymbehandlung bei 37°C oder eine Extraktion mit Natriumnitrit in zwei Arbeitsschritten.

Ein bekanntes, weiteres Verfahren, diese Antigene zu extrahieren, ist eine Behandlung mit hochprozentigem Phenol, z.B. 90 % gesättigtes Phenol (90 % Phenol/10 % Wasser) bei einer Temperatur von 60°C.

Das Erhitzen auf 100°C sowie die Enzymbehandlung zur Antigenfreisetzung können nicht in Gegenwart von spezifischen Antikörpern erfolgen, da diese denaturiert werden. Es muss also eine Umpipettierung erfolgen. Das gleiche gilt für eine hochprozentige Phenolextraktion, die zusätzlich noch eine Zentrifugation erforderlich macht. Die Extraktion mit Natriumnitrit besteht aus zwei separaten Reagenzien und anschliessender Behandlung mit Tris-Puffer.

Dies bedeutet, dass diese bekannten Verfahren äusserst umständlich und zeitaufwendig und somit alles andere als praxiskonform sind.

Im Rahmen der vorliegenden Erfindung wurde nun ein Verfahren zum Freisetzen von Polysaccharid-Antigenen aus Bakterien oder Pilzen in biologischen Proben oder in angezüchteten Kulturen dieser Proben für die nachfolgende immunologische Bestimmung dieser Antigene gefunden, die dadurch gekennzeichnet ist, dass man die biologische Probe oder die angezüchtete Kultur mit einer wässrigen Phenollösung behandelt.

Beim Verfahren gemäss vorliegender Erfindung kann eine 1-10%ige wässrige Phenollösung verwendet werden, wobei eine 2-5%ige wässrige Phenollösung bevorzugt und eine 2%ige wässrige Phenollösung besonders bevorzugt ist.

Als biologische Proben für das Verfahren gemäss vorliegender Erfindung eignen sich Blut, Serum, Plasma, Urin, Faeces oder Sputum.

Es hat sich gezeigt, dass man gemäss dem erfindungsgemässen Verfahren pro 5 Teile biologische Probe bzw. Kultur einen Teil wässrige Phenollösung verwendet.

Beim Verfahren gemäss der vorliegenden Erfindung lässt man die wässrige Phenollösung während 5-30 Minuten, vorzugsweise während 15 Minuten, auf die Probe bzw. Kultur einwirken.

Die bei der Vorbehandlung verwendete Temperatur ist unkritisch. Die Vorbehandlung erfolgt zweckmässig zwischen 15°C und bis knapp vor dem Siedepunkt des Reaktionsgemisches, wobei Raumtemperatur besonders bevorzugt ist.

Zur anschliessenden immunologischen Bestimmung der Polysaccharid-Antigene ist keine Extraktion notwendig, was bedeutet, dass man die immunologische Nachweismethode in dem Milieu durchführen kann, in dem das Freisetzen der Antigene erfolgte.

Die vorerwähnte immunologische Nachweismethode kann in an sich bekannter Weise, insbesondere nach der sogenannten ELISA-Technik erfolgen.

Das Verfahren gemäss vorliegender Erfindung eignet sich hervorragend zum Nachweis von Polysaccharid-Antigenen von Streptokokken, insbesondere von Streptococcus pneumoniae, von Mycobakterien, insbesondere Mycobacterium tuberculosis oder von Enterobakteriaceae, insbesondere Escherichia coli.

Im Rahmen der vorliegenden Erfindung wurde weiterhin gefunden, dass das erfindungsgemässe Verfahren nicht nur weniger arbeitsintensiv ist, sondern in den meisten Fällen auch eine bessere Empfindlichkeit bezüglich der Bestimmung der Polysaccharid-Antigene bietet.

Die nachfolgenden Beispiele illustrieren die Erfindung.

Beispiel 1

A) Methodik des ELISA-Tests.

A.1.) Qualitative Bestimmung von Mykobakterien-Antigenen in kulturell angezüchteten Bakterien und Körperflüssigkeiten mit Mykobakterien-Antikörpern von Kaninchen

In die erforderliche Anzahl Teströhrchen (10 x 25 mm) werden je 0.25 ml Probenmaterial (Bakterien-Suspension, Serum, verflüssigtes Sputum, Urin) pipettiert, dazu werden 0.05 ml des Reaktions reagens (Phenol bzw. $NaNO_2$ + Essigsäure + Trispuffer) bzw. $H_2O$ gegeben. Nach dem Zufügen einer Anti-Mycobakterien-sensibilisierten Polystyrolkugel (∅ = 6.5 mm) pro Teströhrchen wird der Ansatz 30 Minuten bei Raumtemperatur inkubiert. Anschliessend wird ohne Waschvorgang 0.25 ml Kaninchen-Anti-Mycobakterien-Peroxydase-Konjugat (0.1 M Tris-Hydroxymethyl-Aminomethan, pH 7.0 mit 20 % foetalem Kälberserum inaktiviert, 0,05 % Tween 20, und 3 mg/ml Kaninchen-Anti-Mycobakterien-Peroxidase-Konjugat zugefügt. Danach wird der Ansatz 4 h bei Raumtemperatur inkubiert. Nach der Inkubationszeit werden die Polystyrolkugeln im EIA-Washer <Roche> 2-mal mit je 5 ml bidest. Wasser gewaschen. Danach werden in die Teströhrchen je 0.25 ml Substratpuffer für die Aktivitätsbestimmung der Peroxidase (0.1 mol/1 Kaliumcitratpuffer vom pH 5.2 mit 6 mmol/1 $H_2O_2$ und 20 m mol/1 o-Phenylendiamin gegeben und 15 Min. bei Raumtemperatur inkubiert. Zum Abstoppen der peroxidatischen Aktivität sowie zur intensivierung der Farbintensität werden 1.0 ml $(1N)H_2SO_4$ gegeben und innerhalb von 30 Minuten die Extinktion bei der Wellenlänger 492 nm photometrisch gemessen, Z.B. mit dem EIA-Photometer <Roche>.

A.2.) Qualitative Bestimmung von Pneumokokken-Antigenen zu kulturell angezüchteten Bakterien- und Körperflüssigkeiten mit Pneumokokken-Antikörpern von Kaninchen.

In die erforderliche Anzahl Teströhrchen (10 x 25 mm) werden je 0.25 ml Probenmaterial (Bakterien-Suspension bzw. Serum, verflüssigtes Sputum, Urin) bzw. PBS (Leerwert) pipettiert, dazu werden 0.05 ml des Reaktionsreagens (Phenol bzw. $NaNO_2$ + Essigsäure + Trispuffer) bzw. $H_2O$ gegeben. Nach dem Zufügen einer Anti-Pneumokokken-sensibilisierten Polystyrolkugel (∅ = 6.5 mm) pro Teströhrchen wird der Ansatz 15 Minuten bei Raumtemperatur inkubiert. Anschliessend wird ohne Waschvorgang 0.25 ml Kaninchen-Anti-Pneumokokken-Peroxydase-Konjugat (0.1 M Tris-hydroxymethyl-aminomethan, pH 7.0 mit 20 % foetalem Kälberserum inaktiviert, 0.05 % Tween 80 und 0.7 mg/ml Kaninchen-Antipneumokokken-PeroxidaseKonjugat) zugefügt. Danach wird der Ansatz 60 Minuten bei Raumtemperatur inkubiert. Nach der Inkubationszeit werden die Polystyrolkugeln im EIA-Washer <Roche> 2-mal mit je 5 ml bidest. Wasser gewaschen. Danach werden in die Teströhrchen je 0.25 ml Substratpuffer für die Aktivitätsbestimmung der Peroxidase (80 mmol/1 Kaliumcitratpuffer pH 4.25 mit 1.6 mmol/1 $H_2O_2$ und 1 mmol/1 $3,3',5,5'$-Tetramethylbenzidin in 13.4 % DMSO mit 6.6 % 2-Propanol) gegeben und 5 Minuten bei Raumtemperatur gehalten. Zum Abstoppen der peroxidatischen Aktivität sowie zur Intensivierung der Farbenintensität werden 1.0 ml $H_2SO_4$ 1 N gegeben und innerhalb von 30 Minuten die Extinktion bei der Wellenlänge 450 nm photometrisch gemessen, z.B. mit dem EIA-Photometer <Roche>.

A.3.) Qualitative Bestimmung von E. coli-Antigenen in kulturell angezüchteten Bakterien und Körperflüssigkeiten mit E. coli-Antikörpern von Kaninchen

In die erforderliche Anzahl Teströhrchen (10 x 25 mm) werden je 0.25 ml Probenmaterial (Bakterien-Suspension bzw. Serum, Urin) bzw. PBS (Leerwert) pipettiert, dazu werden 0.05 ml des Reaktionsreagens (Phenol bzw. $NaNO_2$ + Essigsäure + Trispuffer) bzw. PBS gegeben. Nach dem Zufügen einer Anti-E.coli sensibilisierten Polystyrolkugel (∅ = 6.5 mm) pro Teströhrchen wird der Ansatz 15 Minuten bei Raumtemperatur inkubiert. Anschliessend wird ohne Waschvorgang 0.25 ml Kaninchen-Anti-E.coli-Peroxydase-Konjugat (0.1 M Trishydroxymethylaminomethan, pH 7.0 mit 20% foetalem Kälberserum inaktiviert 0.05 % Tween 20 und 1mg/ml Kaninchen-Anti-E.coli-Peroxidase-Konjugat) zugefügt. Danach wird der Ansatz 30 Minuten bei RT inkubiert. Nach der Inkubationszeit werden die Polystyrolkugeln im EIA-Washer <Roche> 2-mal mit je 5 ml bidest. Wasser gewaschen. Danach werden in die Teströhrchen je 0.25 ml Substratpuffer für die Aktivitätsbestimmung der Peroxidase (0.1 mol/1 Kaliumcitratpuffer von pH 5.2 mit 6 mmol/1 $H_2O_2$ und 20 m mol/1 o-Phenylendiamin) gegeben und 15 Min. bei Raumtemperatur inkubiert. Zum Abstoppen der peroxidatischen Aktivität sowie zur Intensi- vierung der Farbintensität werden 1.0 ml $H_2SO_4$ (1N) gegeben und innerhalb von 30 Minuten die Extinktion bei der Wellenlänge 492 nm photometrisch gemessen, z.B. mit dem EIA-Photometer <Roche>.

Beispiel 2

B. Freisetzung von Antigenen aus kulturell angezüchteten Bakterien

B.1.) Mycobakterium Calmette Guerin (BCG).

Aus einer frisch gelösten Ampulle des BCG-Impfstoffes des Seruminstituts Bern, wurden mehrere Röhrchen mit Middlebrook-Agar der Firma Difco beimpft. Nach ca. 1-wöchiger Inkubation wurden die Kolonien mit physiologischer NaCl-Lösung abgeschwemmt und auf Mcfarland Dichte von ca. 0.5 eingestellt.

Die Suspension wurde in 5 gleiche Teile aufgeteilt und den verschiedenen Vorbehandlungen unterzogen; danach wurde jeweils ein Einschritt-Enzymimmunoassay durchgeführt. Die Durchführung des Tests erfolgte gemäss Beispiel 1.

Folgende Ergebnisse wurden erhalten:

EP 0 322 549 A2

Mycobakterium Calmette Guerin (BCG)

| Behandlungszeit → | 15 Minuten | | 30 Minuten | |
|---|---|---|---|---|
| Berechnung → <br> Behandlungsart ↓ | OD-Probe − OD-Leerwert | $\dfrac{\text{OD-Probe}}{\text{OD-Leerwert}}$ | OD-Probe − OD-Leerwert | $\dfrac{\text{OD-Probe}}{\text{OD-Leerwert}}$ |
| 250 µl Probe + 50 µl $H_2O$ | 0,794 | 5,1 | 0,842 | 5,8 |
| 250 µl Probe + 50 µl 1 % Phenol | 1,145 | 7,4 | 1,186 | 8,2 |
| 250 µl Probe + 50 µl 2 % Phenol | 1,608 | 9,8 | 1,731 | 10,8 |
| 250 µl Probe + 50 µl 2,5% Phenol | 1,612 | 11,2 | 1,764 | 12,3 |
| 250 µl Probe + 300 µl $H_2O$ | n.d. | n.d. | 0,156 | 2,0 |
| 250 µl Probe + 100 µl $NaNO_2$ 1M <br> + 100 µl Essigs. 1M <br> + 100 µl Trispuffer* | n.d. | n.d. | 0,130 | 1,8 |

* Der Tris-Puffer wurde jeweils 15 Minuten nach Einwirkung der Reagenzien zugefügt.

B.2.) Pneumokokken

Streptococcus pneumoniae wurde über Nacht auf Blutplatten gezüchtet. Danach wurde von den Kolonien eine Suspension in physiologischer NaCl-Lösung mit der Dichte Mcfarland 1.0 hergestellt.

Die Suspension wurde in 5 gleiche Teile portioniert und den verschiedenen Vorbehandlungen unterzogen.

Anschliessend wurde der Einschritt-Enzymimmunoassay durchgeführt. Die Durchführung des Tests erfolgte wie in Beispiel 1.

Folgende Ergebnisse wurden erhalten:


## Pneumokokken - positives Sputum

| Behandlungszeit → | 30 Minuten RT | |
| | | OD-Probe |
| Berechnung → | OD-Probe - OD-Leerwert | OD-Leerwert |
| Behandlungsart ↓ | | |
| 250 µl Probe + 50 µl $H_2O$ | 0,028 | 9,2 |
| 250 µl Probe + 50 µl 5 % Phenol | 2,261 | 26,1 |
| 250 µl Probe + 50 µl $H_2O$ 100°C | 1,232 | 13,7 |
| 250 µl Probe + 1 gH $NaNO_2$ 1M | 1,912 | 18,9 |
| + 1 gH Essigs. 1M | | |
| + 1 gH Tris-Puffer* | | |


\* Der Tris-Puffer wurde jeweils 15 Minuten nach Einwirkung der Reagenzien zugefügt.


B.3.) Escherichia coli

E.coli ATCC 19110 wurde über Nacht auf Blutplatten gezüchtet. Danach wurde von den Kolonien eine Suspension in physiologischer NaCl-Lösung mit der Dichte Mcfarland 1.0 hergestellt. Die Suspension wurde in 4 gleiche Teile portioniert und den ver schiedenen Vorbehandlungen unterzogen. Danach wurde jeweils der Einschritt-Enzymimmunoassay durchgeführt. Durchführung des Tests siehe unter Methodik.

Folgende Ergebnisse wurden erhalten:

6

| Behandlungszeit → | 15 Minuten RT | |
| Berechnung → | OD-Probe - OD-Leerwert | $\dfrac{\text{OD-Probe}}{\text{OD-Leerwert}}$ |
| Behandlungsart ↓ | | |
| 250 µl Probe + 50 µl $H_2O$ | 2,166 | 12,2 |
| 250 µl Probe + 50 µl 2 % Phenol | 1,950 | 11,5 |
| 250 µl Probe + 50 µl 5 % Phenol | 2,096 | 14,8 |
| 250 µl Probe + 50 µl $H_2O$ 100°C | 1,841 | 10,5 |
| 250 µl Probe + 60 µl Regenzgem. (NaNO$_2$. Essigsäure.Tris-Puffer*) | 2,100 | 11,3 |

\* Der Tris-Puffer wurde jeweils 15 Minuten nach Einwirkung der Reagenzien zugefügt.

Beispiel 3

C. Freisetzung von Antigenen aus infektiösem Untersuchungsmaterial

Die weiteren Untersuchungen wurden an Patienten-Material durchgeführt. Der Einschritt EIA-Test wurde gemäss Beispiel 1 durchgeführt. Die Vorbehandlungen erfolgten nach dem beigefügten Schema.

Schema der Parallel-Untersuchungen mit verschiedenen Vorbehandlungen:

| Vorbehandlung→ | Keine | | 100°C | 15 Min | Phenol 2% | | Phenol 5% | | Reagenzien-Gemisch | |
|---|---|---|---|---|---|---|---|---|---|---|
| Ansatz ↓ | L µl | P µl | L µl | P µl | L µl | P µl | L µl | P µl | L µl | P µl |
| Probe | – | 250 | – | 250 | – | 250 | – | 250 | – | 250 |
| PBS | 250 | – | 250 | – | 250 | – | 250 | – | 250 | – |
| H$_2$O bidest | 50 | 50 | 50 | 50 | – | – | – | – | – | – |
| Phenol 2% | – | – | – | – | 50 | 50 | – | – | – | – |
| Phenol 5% | – | – | – | – | – | – | 50 | 50 | – | – |
| NaNO$_2$,1M | – | – | – | – | – | – | – | – | 20 | 20 |
| CH$_3$COOH 0,1M | – | – | – | – | – | – | – | – | 20 | 20 |
| Inkub.15 Min,RT | – | – | – | – | – | – | – | – | x | x |
| Tris-Puffer 0,14 | – | – | – | – | – | – | – | – | 20 | 20 |
| Ab-beladene Kugel | + | + | + | + | + | + | + | + | + | + |
| Vorinkubation | + | + | + | + | + | + | + | + | + | + |
| Konjugat | 250 | 250 | 250 | 250 | 250 | 250 | 250 | 250 | 250 | 250 |
| Immunol.Inkubation | + | + | + | + | + | + | + | + | + | + |
| Waschen | + | + | + | + | + | + | + | + | + | + |
| Substrat-Lösung | 250 | 250 | 250 | 250 | 250 | 250 | 250 | 250 | 250 | 250 |
| Inkubation | + | + | + | + | + | + | + | + | + | + |
| Abstoppreagenz | + | + | + | + | + | + | + | + | + | + |

L = Leerwert (PBS)

P = Probe

EP 0 322 549 A2

Folgende Ergebnisse wurden erhalten:

C.1.) Mycobakterium - positives Sputum

| Berechnung $<br>Behandlungsart ↓ | OD Probe - OD Leerwert | $\dfrac{\text{OD Probe}}{\text{OD Leerwert}}$ |
|---|---|---|
| Negatives Sputum | - 0.068 | 0.7 |
| Sputum unbehandelt | 0.153 | 1.6 |
| Sputum + 2% Phenol | 1.100 | 6.8 |
| Sputum 100°C | 0.535 | 3.3 |
| Sputum + $NaNO_2$ | 0.464 | 2.7 |
| Essigsäure, Tris | | |

C.2.) Pneumokokken - positives Sputum

| Berechnung $<br>Behandlungsart ∘ | OD Probe - OD Leerwert | $\dfrac{\text{OD Probe}}{\text{OD Leerwert}}$ |
|---|---|---|
| Negatives Sputum | - 0.070 | 0.7 |
| Sputum unbehandelt | 1.209 | 7.3 |
| Sputum + 2% Phenol | 1.072 | 6.8 |
| Sputum + 5% Phenol | 1.081 | 8.1 |
| Sputum 100°C | 1.110 | 6.8 |
| Sputum + $NaNO_2$ | 1.052 | 6.2 |
| Essigsäure, Tris | | |

C.3.) E. coli - positive Urine

EP 0 322 549 A2

| Berechnungsart | OD–Probe–OD Leerwert | | | | $\dfrac{\text{OD Probe}}{\text{OD Leerwert}}$ | | | |
|---|---|---|---|---|---|---|---|---|
| Behandlungsart ↓ <br> Urin ↓ | unbehandelt | 2% Phenol | 5% Phenol | 100°C | unbehandelt | 2% Phenol | 5% Phenol | 100° C |
| U 7224 | 0.021 | 0.124 | 0.215 | 0.164 | 1.5 | 3.7 | 3.6 | 4.5 |
| U 7231 | 0.026 | 0.133 | 0.311 | 0.193 | 1.6 | 3.7 | 4.7 | 4.9 |
| U 7261 | 0.093 | 0.160 | 0.233 | 0.068 | 3.1 | 4.7 | 4.2 | 2.4 |
| U 7282 | 0.101 | 0.197 | 0.347 | 0.181 | 2.6 | 4.0 | 4.9 | 3.9 |
| U 7286 | 0.271 | 0.419 | 0.823 | 0.300 | 5.3 | 7.4 | 10.2 | 5.8 |
| U 7293 | 0.057 | 0.112 | 0.190 | 0.250 | 1.9 | 2.9 | 2.8 | 5.3 |

**Ansprüche**

1. Verfahren zum Freisetzen von Polysaccharid-Antigenen aus Bakterien oder Pilzen in biologischen Proben oder in angezüchteten Kulturen dieser Proben für die nachfolgende immunologische Bestimmung dieser Antigene, dadurch gekennzeichnet, dass man die biologische Probe oder die angezüchtete Kultur mit einer wässrigen Phenollösung behandelt.

2. Verfahren nach Anspruch 1, dadurch gekennzeichnet, dass man mit einer 1-10%igen wässrigen Phenollösung arbeitet.

3. Verfahren nach Anspruch 1 oder 2, dadurch gekennzeichnet, dass man mit einer 2-5%igen wässrigen Phenollösung arbeitet.

4. Verfahren nach einem der Ansprüche 1-3, dadurch gekennzeichnet, dass die biologische Probe Blut, Serum, Plasma, Urin, Faeces oder Sputum ist.

5. Verfahren nach einem der Ansprüche 1-4, dadurch gekennzeichnet, dass man mit einer 2%igen wässrigen Phenollösung arbeitet.

6. Verfahren nach einem der Anspüche 1-5, dadurch gekennzeichnet, dass man pro 5 Teile biologische Probe bzw. Kultur einen Teil wässrige Phenollösung verwendet.

7. Verfahren nach einem der Ansprüche 1-6, dadurch gekennzeichnet, dass man die wässrige Phenollösung während 5-30 Minuten auf die Probe bzw. Kultur einwirken lässt.

8. Verfahren nach Anspruch 7, dadurch gekennzeichnet, dass man die wässrige Phenollösung während 15 Minuten auf die Probe bzw. Kultur einwirken lässt.

9. Verfahren zur immunologischen Bestimmung von Polysaccharid-Antigenen aus Bakterien oder Pilzen in einer biologischen Probe oder in einer aus dieser Probe angezüchteten Kultur, dadurch gekennzeichnet, dass man die Probe oder Kultur gemäss einem der Ansprüche 1-8 vorbehandelt und anschliessend die Polysaccharid-Antigene in an sich bekannter Weise immunologisch nachweist.

10. Verfahren nach Anspruch 9, dadurch gekennzeichnet, dass man die Polysaccharid-Antigene von Streptococcen, Mycobacterien oder Enterobacteriacaeen nachweist.

11. Verfahren nach Anspruch 10, dadurch gekennzeichnet, dass man die Polysaccharid-Antigene von Streptococcus pneumoniae, Mycobacterium tuberculosis oder Escherichia coli nachweist.